(19) Europäisches Patentamt
European Patent Office
Office européen des brevets

(11) EP 4 763 235 A1

(12) **EUROPEAN PATENT APPLICATION**

(43) Date of publication:
**24.06.2026 Bulletin 2026/26**

(21) Application number: **24222210.7**

(22) Date of filing: **20.12.2024**

(51) International Patent Classification (IPC):
***A61L 15/24*** *(2006.01)* ***A61L 15/26*** *(2006.01)*
***A61L 15/42*** *(2006.01)*

(52) Cooperative Patent Classification (CPC):
(C-Sets available)
**A61L 15/26; A61L 15/24; A61L 15/42;**
**A61L 15/425; A61L 15/46;** A61L 2300/404;
A61L 2300/606 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ResCure GmbH**
**02625 Bautzen (DE)**

(72) Inventors:
- **Freudenberg, Uwe**
  **01217 Dresden (DE)**
- **Atallah, Passant**
  **01097 Dresden (DE)**
- **Schirmer, Lucas**
  **01127 Dresden (DE)**
- **Kühn, Sebastian**
  **01099 Dresden (DE)**
- **Werner, Carsten**
  **01324 Dresden (DE)**

(74) Representative: **Gleiss Große Schrell und Partner mbB**
**Leitzstraße 45**
**70469 Stuttgart (DE)**

(54) **IMPROVED WOUND DRESSING AND METHOD TO PREPARE IT**

(57) The present invention relates to an improved wound dressing comprising a specific polymeric framework having a coating of a polymer network comprising a polyionic component, in particular a poly-(4-styrenesulfonic acid-co-maleic acid) (PSS-MA) component, covalently bonded to at least one of an uncharged polymer component and a non-polymeric crosslinker component thereby forming the polymer network, wherein the polyionic component, in particular the PSS-MA component, comprises sulfonate and/or sulfate groups, and a method to prepare it.

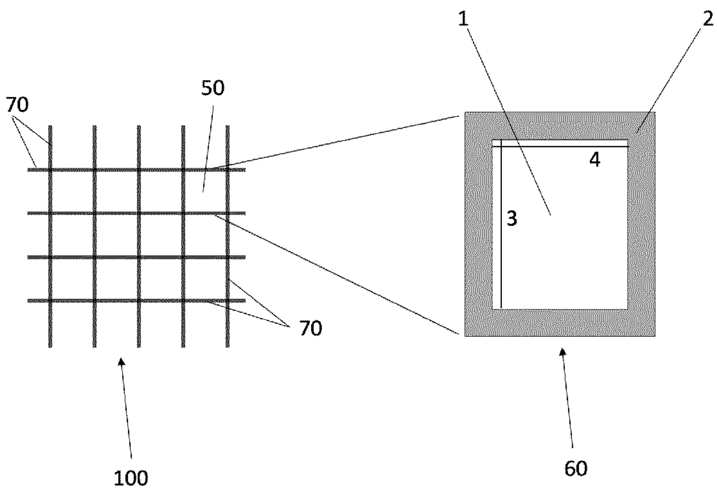


Figure 1



Processed by Luminess, 75001 PARIS (FR)

(Cont. next page)

(52) Cooperative Patent Classification (CPC): (Cont.)

C-Sets
**A61L 15/24, C08L 35/00;**
**A61L 15/26, C08L 71/02**

## Description

The present invention relates to a wound dressing comprising a specific flat polymeric framework having a coating of a polymer network comprising at least one polyionic component, in particular at least one poly-(4-styrenesulfonic acid-co-maleic acid) (PSS-MA) component, covalently bonded to at least one of an uncharged polymer component and a non-polymeric crosslinking component thereby forming the polymer network, wherein the polyionic component, in particular the PSS-MA component, comprises sulfonate, sulfate or sulfonate and sulfate groups, and a method to prepare it.

In Germany, more than 2.7 million patients suffer from chronic, non-healing wounds. The treatment of these wounds can take months to years and requires frequent visits to the doctor and nursing care. With an aging population and increasing incidence of diabetes and obesity, many more patients will suffer from hard-to-heal wounds in the future. The main cause of delayed wound healing is a prolonged and intensified inflammatory phase, which prevents the onset of healing. Accordingly, a multitude of various therapeutic applications have been developed so as to effectively cure wounds.

EP 1 718 257 B1 discloses a multi layered wound dressing for highly exudating wounds, which includes a transmission layer having high moisture vapor transmission rate, absorbent core, and a wound contacting layer.

EP 1 988 909 B 1 discloses a hydrogel composition or aqueous solution useful in the treatment of inflammation of the skin or mucosa and as a dermatological product, which comprises poly(ethylene glycol) having specific molecular weight.

EP 2 736 486 A1 discloses a composition useful for treating a wound, which comprises a pharmaceutically acceptable carrier, an effective amount of an active ingredient of inorganic solids, and an antimicrobial biguanide compound.

EP 2 742 997 B1 discloses a carrier used for, for instance, inflammatory cytokine adsorption, which comprises high molecular com-pound(s) having two or more repeating units containing aromatic ring which is covalently bonded through specific structure.

WO 2021/198464 A1 discloses a hydrogel-based wound dressing formulation useful for treating wounds, which comprises a copolymer of monomers with at least one covalently linked multifunctional group, and a source of nitrite and/or nitrate.

WO 2021/212154 A1 discloses a wound dressing useful for treating wounds, which comprises a substrate having first surface and second surface comprising a sulfonated polymeric layer containing sulfonated polymer.

The known technologies often use specific substances which are classified as pharmaceuticals and thus frequently cause from side-effects and need to be admitted in costly and time-consuming proceedings by the competent drug authorities prior to use. Other technologies do not exert the desired wound healing effects in a satisfactory manner or are complicated and expensive in production, structure and/or use.

Thus, there is an urgent need to provide means and methods to improve wound healing, in particular to allow a rapid wound healing, in particular with a reduced inflammatory phase, in particular in a cost-effective and simple manner, in particular wherein the means and methods do not rely on the use of pharmaceutically active agents.

The technical problem underlying the present invention therefore is to overcome the aforementioned disadvantages.

Thus, the technical problem underlying the present invention is in particular to provide means and methods to improve the wound healing process in subjects in need thereof, in particular humans and animals, in particular means and methods which allow a faster wound healing and reduce the duration and/or intensity of the inflammatory phase, in particular wherein the means and methods do not rely on the use of pharmaceutically active agents. Preferably, such means and methods should reduce pain and discomfort of the patients during its use and also during and after removal of the wound dressing from the wound.

The technical problem is solved by the teaching of the present invention, in particular the teaching as specified in the independent and dependent claims as well as in the present description.

Thus, the present invention, in particular, provides a wound dressing comprising a polymeric flat framework having a coating of a polymer network, the polymer network comprising at least one polyionic polymer component covalently bonded to at least one of at least one uncharged polymer component and at least one non-polymeric cross-linking component, thereby forming the polymer network, wherein the at least one polyionic polymer component comprises sulfate, sulfonate or sulfate and sulfonate groups, wherein the polymeric flat framework has a top and a bottom surface and is composed of a multitude of openings and a multitude of frames surrounding the openings and wherein the ratio of the area of the openings to the area of the frame surfaces, each in the top surface of the framework, is from 0.5 to 6, and the number of sulfate, sulfonate or sulfate and sulfonate groups/total area of frame surfaces is from 100 to 950 nmol/$cm^2$, in particular of 200 to 400 nmol/$cm^2$, in particular 350 to 800 nmol/$cm^2$.

Preferably, the ratio of the area of the openings to the area of the frame surfaces, each in the top surface of the framework, is in particular from 1.5 to 2.5.

Preferably, the ratio of the area of the openings to the area of the frame surfaces, each in the top surface of the framework, is in particular from 1.6 to 2.2.

Preferably, the number of sulfate, sulfonate or sulfate and sulfonate groups/total area of frame surfaces is from 200 to 400 nmol/cm$^2$.

Preferably, the number of sulfate, sulfonate or sulfate and sulfonate groups/total area of frame surfaces is from 350 to 800 nmol/cm$^2$.

Thus, the present invention in particular provides a wound dressing comprising a polymeric framework having a coating of a polymer network, the polymer network comprising at least one polyionic polymer component (in the following also termed PIP), in particular at least one poly-(4-styrenesulfonic acid-co-maleic acid) (in the following also termed PSS-MA) component, covalently bonded to at least one of at least one uncharged polymer component and at least one non-polymeric crosslinking component thereby forming the polymer network, wherein the PPI-component comprises sulfate or sulfonate groups or both, in particular wherein the PSS-MA component comprises sulfonate groups.

Thus, the present invention in particular provides a wound dressing comprising a polymeric framework having a coating of a polymer network, the polymer network comprising at least one polyionic polymer component, in particular PSS-MA component, covalently bonded to at least one of at least one uncharged polymer component and at least one non-polymeric crosslinking component thereby forming the polymer network, wherein the polyionic polymer component, in particular PSS-MA component, comprises sulfonate or sulfate groups or both, which means that the at least one PIP, in particular PSS-MA, component is bonded to the at least one uncharged polymer component or to the at least one non-polymeric crosslinking component or to both components.

Thus, the present invention in particular provides a wound dressing comprising a polymeric framework having a coating of a polymer network, the polymer network comprising at least one polyionic polymer component, in particular at least one PSS-MA component, covalently bonded to at least one of at least one uncharged polymer component and at least one non-polymeric crosslinking component thereby forming the polymer network, wherein the PIP, in particular PSS-MA, component comprises sulfonate or sulfate groups or both, which means that the at least one polyionic polymer component, in particular at least one PSS-MA component is bonded to the at least one uncharged polymer component and/or to the at least one non-polymeric crosslinking component.

The PIP component in the polymer network comprises sulfonate, sulfate or sulfonate and sulfate groups, that means free, unbound and ionizable, functional groups which are preferably negatively charged, in particular under physiological conditions, that means under the condition of being applied to a wound, and which are, thus, not covalently bond to other residues or entities of the polymeric framework or the polymer network.

In a preferred embodiment, the PIP component is selected from the group consisting of poly-(4-styrenesulfonic acid-co-maleic acid) (PSS-MA), poly(acrylic acid-co-2-acrylamido methylsulfonate), poly(acrylic acid-co-2-acrylamido ethanesulfonate), poly(acrylic acid-co-2-acrylamido ethanesulfate), poly(acrylic acid-co-4-(acrylamidomethyl)benzenesulfonate), poly(methyl methacrylate)-styrene sulfonate terpolymers, poly (sulfonated styrene-co-methyl methacrylate), poly (sulfonated styrene-co-acrylic acid-co-methyl methacrylate), sulfated poly(vinyl alcohol-co-acrylic acid), and sulfonated poly(glutamic acid).

In a preferred embodiment, the PIP component is poly-(4-styrenesulfonic acid-co-maleic acid) (PSS-MA).

The PSS-MA component in the polymer network comprises sulfonate groups, that means free, unbound and ionizable, functional groups which are preferably negatively charged, in particular under physiological conditions, that means under the condition of being applied to a wound, and which are, thus, not covalently bond to other residues or entities of the polymeric framework or the polymer network.

Without being bound by theory, the sulfonate and sulfate groups in the polymer network are preferably essentially in deprotonated form. These deprotonated sulfonate and sulfate groups can undergo charge compensation or form charge interactions by counterions and/or proteins. The sulfonate groups may in some embodiments be in protonated form.

These sulfonate and sulfate groups are accordingly and preferably in the polymer network present in form of anionic groups and are thus able to interact based on the electrical charge, with cationic groups, and therefore are capable to bind and release positively charged molecules, in particular chemokines. According to the invention, these sulfonate and sulfate groups are not covalently bonded to the uncharged polymer component, in particular not to the preferred poly(ethylene glycol) (PEG) component. According to the invention, these sulfonate and sulfate groups are not covalently bonded to the non-polymeric crosslinking component. The PIP, in particular PSS-MA, used to prepare the polymer network comprises also carboxylate or carboxyl groups, which, however, are primarily used to provide covalent bonds to other components of the polymer network, in particular to the uncharged polymer component and/or the non-polymeric crosslinking component. Thus, at least most of the carboxylate or carboxyl groups of the PIP, in particular PSS-MA, component, are no free groups but participate in covalently binding components of the polymer network to each other.

Thus, the present invention provides a wound dressing comprising, in particular consisting of, a polymeric framework and a coating thereof, which coating is comprising, in particular consisting of, a polymer network as specified herein. The polymer network preferably coats (hereinafter also referred to as "surrounds") the polymeric framework completely or partially, that means the polymer network constitutes a complete or partial, preferably complete, coating of

the surface of the polymeric framework, in particular of its plates and/or and/or mesh or foam structures.

The polymeric flat framework (hereinafter also termed to be a "carrier" or "support") may be a foam, in particular polymeric foam, a textile or fabric, in particular a nonwoven fabric or a woven fabric, a knitted fabric, a mesh, a perforated film, a perforated membrane or a perforated plate. The textile or fabric may be in the form of a mesh. Thus, the mesh may be a textile mesh or a fabric mesh.

Preferably, the framework is a mesh, a perforated film, a perforated membrane or a perforated plate.

Preferably, the framework is a mesh, in particular a fibre mesh, a mesh netting, a grid or a matrix.

Preferably, the framework is a perforated film, a perforated membrane or a perforated plate.

Preferably, the framework is a foam, in particular polymeric foam.

The framework may be in the form of frames and openings, wherein the frames may be in the form of struts, threads, filaments or fibres. The framework may also be in form of a film, membrane or plate with perforations, the perforations being the openings and the frames being those parts of the plate, film or membrane which surround the perforations and thus define their shape and size.

Thus, all frames of a framework build up the framework.

Preferably, the mesh is composed of a multitude of openings, wherein each opening is surrounded by a frame, which frame may be in the form of struts, filaments, threads or fibres.

Preferably, the frame of the mesh may have a circular, oval, rectangular, in particular square, or polygonal cross-sectional outline, in particular shape.

Preferably, the plate, film or membrane is composed of a multitude of openings, which are located in a plate body, a film body or a membrane body, wherein each opening is surrounded by a frame, which frame may be an integral part of the body of the plate, film or membrane and surrounds the openings.

Preferably, the framework, in particular mesh, comprises from 2 to 10 000, preferably from 100 to 9000, preferably from 300 to 8000 preferably from 500 to 7000 openings.

Preferably, the framework, in particular mesh, comprises from 2 to 200, preferably from 10 to 150, preferably from 30 to 100, preferably from 50 to 90 openings per $cm^2$ of the top surface of the mesh.

Preferably, the perforated plate, film or membrane comprises from 2 to 10 000, preferably from 100 to 9000, preferably from 300 to 8000 preferably from 500 to 7000 openings.

Preferably, the perforated plate, film or membrane comprises from 2 to 200, preferably from 10 to 150, preferably from 30 to 100, preferably from 50 to 90 openings per $cm^2$ of the top surface of the. plate, film or membrane.

Preferably, the framework, in particular mesh, plate, film or membrane, comprises the openings in homogenous distribution over the top surface of the framework.

Preferably, the framework, in particular mesh, plate, film or membrane, comprises the openings in equal distance to each other on the top surface of the framework.

The present invention in a preferred embodiment relates to a wound dressing, wherein the framework has an opening size from 500 $\mu$m to 2 mm, preferably 600 $\mu$m to 1,5 mm, preferably 700 $\mu$m to 1 mm. The opening size as referred to herein is the smallest diameter of the opening.

In a preferred embodiment, all openings in the framework have the same size, the same outline, in particular shape, preferably the same size and outline, in particular shape.

The framework of the dressing may comprise frames of similar or different sizes and structure. Accordingly, the framework may thus form round, rectangular or irregular shaped openings such as they are characteristics for meshes, foams or other frameworks.

Preferably, the openings have a circular, oval, rectangular, in particular square, or polygonal outline, in particular shape.

The openings may be in the form of pores.

The present invention in a preferred embodiment relates to a wound dressing, wherein the polymeric framework is made of a synthetic polymer, in particular polyester, polyamide, cellulose, polyurethane, silicone polymer or polyethylene.

In a preferred embodiment, the polymeric framework is a polyurethane foam.

In a preferred embodiment, the polymeric framework is a mesh. Preferably, the mesh is a polyester mesh.

The polymer-coated wound dressing of the present invention protects the wound from physical influences of an optionally applied secondary wound dressing and minimizes irritation of the wound during dressing change. As a result, it leads to relief of patient pain and discomfort, and minimizes trauma to the wound and surrounding skin during dressing changes. The main effect of the polymeric framework is its function as a wound spacer being achieved by creating a mechanical barrier between the wound and an additionally and optionally applied secondary wound dressing. The polymeric framework protects the wound from sticking to the secondary wound dressing, thereby enabling atraumatic wound dressing changes and, at the same time, its meshed or foam structure also ensures the removal of excess wound exudate and its absorption by the secondary wound dressing or the polymeric foam-framework itself.

The wound dressing very advantageously comprises a unique polymer coating, that means the polymer network,

on the polymeric framework, which promotes the causal resolution of the chronic inflammatory process, thus supporting wound closure and enabling a rapid wound healing. This shortens the duration of inflammation, pain and discomfort and prevents further damage to the surrounding skin and other tissues as well as a potential consequential damage such as an amputation.

The polymer coating of the polymeric framework, which comprises, in particular consists of, at least one PIP-component, in particular PSS-MA component, and covalently cross-linked thereto at least one of at least one uncharged polymer component, in particular a star-PEG, and at least one non-polymeric crosslinking component ensures hydrophilization of the polymeric framework through water retention in the polymer network and prevents the wound from sticking to the framework. In addition, the at least one PIP-component, in particular PSS-MA component, covalently bound in the polymer coating that means the polymer network, promotes wound healing by physically binding of pro-inflammatory substances, in particular chemokines, and withdrawing them from the wound area. The chronic inflammatory process can thus be stopped and the transition to secondary wound healing thus enabled. Thus, a physical adsorption process takes place that brings about this effect and is based on charge interactions between the negatively charged sulfonate of the PIP-component, in particular PSS-MA component, and positively charged amino acid residues of the pro-inflammatory chemokines.

The wound dressing very advantageously can easily be removed from the wound after its application without causing small or severe damages to the tissue. The healed wound is thus not or hardly being damaged during removal of the wound dressing and the patient experiences no pain during removal. Advantageously, the polymer network coating thus prevents adherence of the dressing to the wound and subsequent trauma to the wound bed. This finding improves the comfort for the patient and thus patient compliance to the wound dressing. In view of the anionic groups present in the polymer coating, which are expected to interact with the proteins around and in the wound tissue, this finding is surprising.

The main effect of the present wound dressing as a polymer-coated wound spacer grid is therefore a) in the mechanical barrier function to an optional secondary wound dressing preventing the wound from sticking to the secondary wound dressing, b) in enabling fluid management of the wound, that means excess wound secretion (i.e. wound exudate) can be drained off and absorbed by a secondary wound dressing, and c) the physical process of binding excess, pro-inflammatory substances in particular chemokines, and removing them from the wound area, brought about by the PIP-component, in particular PSS-MA component, covalently bound in the polymer coating thereby providing a covalently crosslinked, hydratable polymer network.

According to one embodiment of the invention, the at least one uncharged polymer component is covalently bonded to the PIP-component, in particular at least one PSS-MA component, in particular directly or by means of a crosslinking component. The uncharged polymer component and the PIP-component, in particular PSS-MA component, covalently bonded thereto thus together, optionally with the additional presence of the non-polymeric crosslinking component, form the polymer network.

According to the invention, in a further embodiment, the at least one non-polymeric crosslinking component is covalently bonded to the at least one PIP-component, in particular at least PSS-MA component, in particular directly. The at least one non-polymeric crosslinking component and the PIP-component, in particular the PSS-MA component covalently bonded thereto, thus, together form the polymer network.

The invention therefore provides in particular that the at least one PIP-component, in particular PSS-MA component, is covalently bonded directly or via at least one crosslinking component to the at least one non-charged polymer component, forming the polymer network, or, in a further embodiment, that the at least one PSS-MA component, is bonded to the at least one crosslinking component, forming the polymer network.

In a preferred embodiment, PSS-MA may have a MW of 10 000 to 40 000 g/mol, in particular 20 000 to 30 000 g /mol, preferably 20 000 to 21 000 g/mol.

In a preferred embodiment, the PSS-MA is a copolymer with a molar ratio from 5:1 to 1:1, in particular 3:1 to 1:1 of 4-styrenesulfonic acid: maleic acid.

The PIP, in particular PSS-MA, used preferably for the preparation of the polymer network comprises, in addition to sulfonate groups, carboxylate or carboxyl groups, with which the PIP-component, in particular PSS-MA, is covalently bonded to the uncharged polymer and/or the non-polymeric crosslinking component used to form the polymer network, such that the network is formed having at least one PIP-component, in particular PSS-MA component, crosslinked via at least one uncharged polymer component or at least one non-polymeric crosslinking component or crosslinked via both to form a network.

In a particularly preferred embodiment, the at least one uncharged polymer component is selected from the group consisting of poly(ethylene glycol) (PEG) component, poly(2-oxazoline) (POX) component, polyvinylpyrrolidone (PVP) component, poly(vinyl alcohol) (PVA) component, polyacrylamide (PAM) component, and combinations thereof.

The uncharged polymer may be linear or branched, in particular multi-arm, that is, this comprises several branched chains in a preferred embodiment of the present invention. In particular, the uncharged polymer is star-shaped, that is, multi-arm with a centre from which a plurality of chains, in particular four or eight chains, in particular four chains of equal length, branch off from corresponding repeating units.

In a preferred embodiment, the at least one uncharged polymer has an average molecular weight of from 5 000 to 25 000, in particular from 10 000 to 19 000 Da.

In a preferred embodiment, the repeating unit of the at least one uncharged polymer component has an average molecular weight of from 30 to 55, in particular from 40 to 50 Da.

In a particularly preferred embodiment, the at least one uncharged polymer component is a linear uncharged polymer component.

In a particularly preferred embodiment, the at least one uncharged polymer component is a branched, in particular multi-arm, uncharged polymer component.

In a particularly preferred embodiment, the at least one multi-arm uncharged polymer component is a four-armed or eight-arm uncharged polymer component.

In a particularly preferred embodiment, the at least one multi-arm uncharged polymer component is a PEG component.

In a particularly preferred embodiment, the at least one uncharged polymer component is a poly(ethylene glycol) component, particularly a linear or multi-arm poly(ethylene glycol) component.

In a particularly preferred embodiment, the at least one multi-arm uncharged polymer component is a four-arm poly(ethylene glycol) component.

In another preferred embodiment, the at least one multi-arm uncharged polymer component is an eight-arm poly(ethylene glycol) component.

In a particularly preferred embodiment, the at least one multi-arm uncharged polymer component is a four-arm PEG. A multi-arm PEG, in particular a four-arm PEG, is hereinafter also called a starPEG.

In a preferred embodiment, starPEG may have a molecular weight (MW) from 6 000 to 20 000 g/mol, preferably 10 000 to 16 000 g/mol.

In a preferred embodiment of the present invention, the uncharged polymer used to prepare the polymer network has a functional group, in particular an end group, at the respective free end of the polymer selected from the group consisting of amino group, thiol group, maleimide group, vinylsulfone group, acrylate group, carboxyl group and combinations thereof. The uncharged polymer is thus functionalized, in particular end group functionalized. Via these reactive functional groups, in particular end groups, the at least one uncharged polymer is preferably linked to the at least one PIP-component, in particular PSS-MA, thus forming the polymer network. The at least two functional groups of the uncharged polymer may be the same or different, in particular they are the same.

In a particularly preferred embodiment, the uncharged polymer used for preparing the polymer network according to the invention has at least two amino groups, in particular terminal amino groups.

In a particularly preferred embodiment, the uncharged polymer used for the production of the polymer network according to the invention has at least two carboxyl groups, in particular terminal carboxyl groups.

In a particularly preferred embodiment, the uncharged polymer, which is in particular end group functionalized with one carboxyl group each, is a multi-armed, in particular eight-armed, PEG.

In a particularly preferred embodiment, the uncharged polymer, which is in particular end-group-functionalized with one amino group in each case, is a multi-armed, in particular four-armed, PEG.

In a preferred embodiment of the present invention, the non-polymeric crosslinking component used for the preparation of the polymer network according to the invention has at least two functional groups suitable for forming a covalent bond to the PIP-component, in particular PSS-MA component.

In preferred embodiments of the present invention, the non-polymeric crosslinking molecule used to prepare the polymer network has at least two functional groups, in particular end groups, selected from the group consisting of amino group, thiol group, maleimide group, vinylsulfone group, acrylate group, carboxyl group, hydroxylated aromatic group and combinations thereof. The non-polymeric crosslinking molecule is thus functionalized, particularly end group functionalized. Via these reactive functional groups, in particular end groups, the at least one non-polymeric crosslinking molecule is preferentially linked to the at least one PIP-component, in particular PSS-MA, thus forming the polymer network. The at least two functional groups of the non-polymeric crosslinking molecule may be the same or different, in particular they are the same.

In a preferred embodiment, the crosslinking molecule is a non-polymeric bifunctional crosslinking molecule.

In a preferred embodiment, the crosslinking molecule has at least two amino groups. In a particularly preferred embodiment, the crosslinking molecule having at least two amino groups is selected from the group consisting of ethylenediamine, propylenediamine (1,3-diaminopropane), butane-1,4-diamine, pentane-1,5-diamine (cadaverines), hexamethylene-1,6-diamine, and combinations thereof.

In a preferred embodiment, the crosslinking molecule has at least two carboxyl groups. In a particularly preferred embodiment, the crosslinking molecule having at least two carboxyl groups is selected from the group consisting of oxalic acid, malonic acid, succinic acid, glutaric acid, adipic acid, pimelic acid, and combinations thereof.

In a particularly preferred embodiment, the crosslinking molecule is a molecule having at least two different functional groups, in particular at least two functional groups capable of crosslinking the PIP-component, in particular PSS-MA component, and the uncharged polymer component, in particular N-(2-aminoethyl) maleimide.

In a preferred embodiment, the at least one non-polymeric crosslinking component is selected from the group consisting of ethylenediamine, propylenediamine (1,3-diaminopropane), butane-1,4-diamine, pentane-1,5-diamine (cadaverine), hexamethylene-1,6-diamine oxalic acid, malonic acid, succinic acid, glutaric acid, adipic acid, pimelic acid, N-(2-aminoethyl) maleimide, an enzymatically cleavable peptide and combinations thereof.

In a preferred embodiment of the present invention, the uncharged polymer components and the PIP-component, in particular PSS-MA components, are covalently bonded by means of at least one non-polymeric crosslinking component, in particular an enzymatically cleavable peptide.

In a preferred embodiment, the starPEG may be a starPEG peptide conjugate, preferably wherein the peptides carry active thiol groups on the cysteine for crosslinking.

Preferably, for providing enzymatically cleavable polymer network, starPEG-mal can be pre-functionalized with enzymatic cleavable peptides carrying cysteine with free thiol groups at the end of the peptide sequences allowing for the crosslinking via reaction with maleimide pre-functionalized PIP-component, in particular PSS-MA.

In a particularly preferred embodiment, the crosslinking molecule is in particular an enzymatically cleavable sequence, in particular a matrix metalloproteases (MMPs)-responsive element, cathepsin-responsive element, elastase-responsive element, blood coagulation enzyme-responsive element, for example thrombin-responsive GGF-pipecolic acid, FXa-responsive element, kallikrein-responsive element or bacterial protease-responsive element, for example aureolysin-responsive or elastase-responsive element, or the protease IV-responsive sequence.

In a preferred embodiment, the enzymatically cleavable sequences are each flanked at the C- and N-terminal ends by a cysteine. The PIP-component, in particular PSS-MA, and the uncharged polymer or at least two PIP-components, in particular PSS-MA, are crosslinked via the respective cysteine.

In a preferred embodiment, the PIP-component, in particular PSS-MA component, used for the preparation of the polymer network according to the invention is covalently linked to a non-polymeric crosslinking component, in particular peptide.

In a preferred embodiment, the uncharged polymer component preferably used for the preparation of the polymer network according to the invention and the PIP-component, in particular PSS-MA component, are covalently linked to each other by means of a non-polymeric crosslinking component, in particular peptide.

In a preferred embodiment, the non-polymeric crosslinking component and the PIP-component, in particular PSS-MA, component are covalently linked to each other via at least one amide bond.

In a preferred embodiment, the uncharged polymer component and the PIP-component, in particular PSS-MA component, are covalently linked to each other via at least one amide bond by means of a non-polymeric crosslinking component.

In another preferred embodiment, the uncharged polymer component and the PIP-component, in particular PSS-MA component, used to prepare the polymer network of the invention are directly covalently bonded to one another, in particular by means of an amide bond, thiol-amine bond, disulfide bond, thioether bond, thiol-maleimide bond, preferably bioorthogonal thioether bond obtainable by thiol-maleimide, thiol-vinyl sulfone or thiol-acrylate reaction.

In a preferred embodiment, the uncharged polymer component, in particular uncharged polymer component containing amino groups, and the PIP-component, in particular PSS-MA component, containing carboxyl groups, used for the preparation of the polymer network according to the invention are covalently bonded directly to one another by means of an amide bond. In a preferred embodiment, the amide bond is facilitated by means of 1-ethyl-3-(3-dimethylaminopropyl) carbodiimide/N-hydroxysulfosuccinimide (EDC/sNHS) activation at the carboxyl groups of the PIP-component, in particular PSS-MA component.

In a further preferred embodiment, the non-polymeric crosslinking component used for the preparation of the polymer network according to the invention, and the PIP-component, in particular PSS-MA component, are directly covalently bonded to each other, in particular by means of an amide bond, thiol-amine bond, disulfide bond, thioether bond, thiol-maleimide bond, preferably bioorthogonal thioether bond obtainable by thiol-maleimide, thiol-vinyl sulfone or thiol-acrylate reaction.

In a preferred embodiment, the non-polymeric crosslinking component, in particular non-polymeric crosslinking component containing amino groups, and the PSS-MA containing carboxyl groups used for the preparation of the polymer network according to the invention are covalently bonded directly to one another by means of an amide bond. In a preferred embodiment, the amide bond is facilitated by means of 1-ethyl-3-(3-dimethylaminopropyl) carbodiimide/N-hydroxysulfosuccinimide (EDC/sNHS) activation at the carboxyl groups of the PSS-MA component.

In a preferred embodiment, the polymer network comprises at least two, in particular two, different non-polymeric crosslinking components. In a particularly preferred embodiment, the polymer network has a non-polymeric crosslinking component which has at least two carboxyl groups, in particular is end-group-functionalized with one carboxyl group in each case, and a non-polymeric crosslinking component which has at least two amino groups, in particular is end-group-functionalized with one amino group in each case. Preferably, the non-polymeric crosslinking component having at least two carboxyl groups can be linked via at least one amide bond to the non-polymeric crosslinking component preferably

having amino groups, and the non-polymeric crosslinking PIP-component, in particular component, having at least two amino groups can be linked via at least one amide bond to the PIP-component, in particular PSS-MA component, having carboxyl groups, wherein in a preferred embodiment the amide bond is enabled by means of 1-ethyl-3-(3-dimethylaminopropyl)carbodiimide/N-hydroxysulfosuccinimide (EDC/sNHS) activation at the carboxyl groups of the non-polymeric crosslinking molecule and the PIP-component, in particular PSS-MA component.

In a preferred embodiment, the polymer network comprises at least two, in particular two, different uncharged polymer components. In a particularly preferred embodiment, the polymer network comprises an uncharged polymer component which has at least two carboxyl groups, in particular is end-group-functionalized with one carboxyl group in each case, and an uncharged polymer component which comprises at least two amino groups, in particular is end-group-functionalized with one amino group in each case. Preferably, the uncharged polymer component having at least two carboxyl groups can be connected via at least one amide bond to the uncharged polymer component preferably having amino groups, and the uncharged polymer component having at least two amino groups can be connected via at least one amide bond to the PIP-component, in particular PSS-MA component, having carboxyl groups, wherein in a preferred embodiment the amide bond is enabled by means of 1-ethyl-3-(3-dimethylaminopropyl)carbodiimide/N-hydroxysulfosuccinimide (EDC/sNHS) activation at the carboxyl groups of the uncharged polymer component and the PIP-component, in particular PSS-MA component.

In a preferred embodiment, the polymer network comprises an uncharged polymer component. In a particularly preferred embodiment, the polymer network has an uncharged polymer component that has at least two amino groups, in particular is end-group functionalized with one amino group each. Preferably, the uncharged polymer component having at least two amino groups can be linked via at least one amide bond to the PIP-component, in particular PSS-MA, having carboxyl groups, wherein in a preferred embodiment the amide bond is enabled by means of 1-ethyl-3-(3-dimethylaminopropyl) carbodiimide/N-hydroxysulfosuccinimide (EDC/sNHS) activation at the carboxyl groups of the PIP-component, in particular PSS-MA component.

In a particularly preferred embodiment, the polymer network, in particular in the swollen state, has sulfonate groups in a concentration of at least 0.1 mmol/l, in particular at least 1 mmol/l, in particular at least 10 mmol/l, in particular at least 100 mmol/l, in particular 0.1 to 800 mmol/l, in particular 10 to 800 mmol/l, in particular 1 to 100 mmol/l, in particular 20 to 500 mmol/l, in particular 20 to 200 mmol/l, in particular 50 to 200 mmol/l, in the volume of the polymer network present in the swollen state.

In a preferred embodiment, the polymer network has an average molecular mesh size of 5 to 30 nm, in particular 9 to 16 nm.

In a preferred embodiment, the polymer network has in the process of its synthesis a solid content from 0,4 to 10, preferably from 1 to 9, preferably from 5 to 8, preferably 6 to 7 weight % (weight of polymer components based on overall weight).

In a particularly preferred embodiment, the coating is present in a swollen state, in particular in aqueous solution, in particular water, or buffered aqueous solution, in particular PBS, swollen form.

In a preferred embodiment, the polymer network, in particular the wound dressing, does not contain potassium ions, preferably no metal ions.

In a preferred embodiment, the polymer network, in particular the wound dressing, contains sodium ions, preferably contains sodium ions as the only metal ions.

In a preferred embodiment, the coating made from the polymer network has a thickness of at least 1 $\mu$m, preferably at least 3 $\mu$m.

In a preferred embodiment, the coating made from the polymer network has a thickness from 0.5 to 20, preferably 1 to 4, preferably 2 to 8, preferably 0.05 to 0.2, preferably 10 to 50 $\mu$m. (

In a preferred embodiment the present wound dressing is in the form of a patch, a wound plaster or bandage.

The present invention in a preferred embodiment relates to a wound dressing, wherein the wound dressing comprises a swellable polymer, in particular CMC (carboxymethyl cellulose), pectin or gelatin.

The present wound dressing in a preferred embodiment is biocompatible, but not bioabsorbable or degradable.

In a preferred embodiment, the polymer network is a synthetic polymer network.

In a preferred embodiment, the polymer network is hydratable.

In a preferred embodiment the present wound dressing does not contain any pharmaceutical substance.

In a preferred embodiment the present wound dressing does not contain any nitrates or nitrites or sources thereof.

In a preferred embodiment the present wound dressing does not contain any other components or substances, in particular no polymers, except the polymeric framework and its coating made from the polymer network.

In a preferred embodiment, the polymer network of the present invention is the only polymer network present in the present wound dressing.

In a preferred embodiment, the present wound dressing contains one, in particular one single, polymer network.

In a preferred embodiment, the present wound dressing contains one, in particular one single, polymeric

framework.

In a preferred embodiment the present wound dressing comprises at least one anti-microbial substance.

In a preferred embodiment the present wound dressing is non-adhesive.

In a preferred embodiment the present wound dressing comprises at least adhesive edge, preferably along at least one peripheral edge of the wound dressing.

In a preferred embodiment the present wound dressing is attached or attachable to a secondary wound dressing, preferably wherein the secondary wound dressing is attachable or attached distal to the wound that means the present wound dressing being located in between the wound and the secondary wound dressing.

The present invention in particular also provides a wound dressing comprising a polymeric flat framework having a coating of a polymer network, the polymer network comprising at least one polyionic polymer component covalently bonded to at least one of at least one uncharged polymer component and at least one non-polymeric cross-linking component thereby forming the polymer network, wherein the at least one polyionic polymer component comprises sulfate, sulfonate or sulfate and sulfonate groups, wherein the polymeric flat framework has a top and a bottom surface and is composed of a multitude of openings and a multitude of frames surrounding the openings and wherein the ratio of the area of the openings to the area of the frame surfaces, each in the top surface of the framework, is from 0.5 to 6, in particular 1.5 to 2.5, in particular 1.6 to 2.2 and the number of sulfate, sulfonate or sulfate and sulfonate groups/opening is 1 to 8 in nmol/opening.

In a preferred embodiment, the number of sulfate, sulfonate or sulfate and sulfonate groups/opening is in particular 2 to 4 in nmol/opening.

In a preferred embodiment, the number of sulfate, sulfonate or sulfate and sulfonate groups/opening is in particular 5 to 8 in nmol/opening.

The present invention in particular also provides a wound dressing comprising a polymeric flat framework having a coating of a polymer network, the polymer network comprising at least one polyionic polymer component covalently bonded to at least one of at least one uncharged polymer component and at least one non-polymeric cross-linking component thereby forming the polymer network, wherein the at least one polyionic polymer component comprises sulfate, sulfonate or sulfate and sulfonate groups, wherein the polymeric flat framework has a top and a bottom surface and is composed of a multitude of openings and a multitude of frames surrounding the openings and wherein the ratio of the area of the openings to the area of the frame surfaces, each in the top surface of the framework, is from 0.5 to 6, in particular 1.5 to 2.5, in particular 1.6 to 2.2 and the number of sulfate, sulfonate or sulfate and sulfonate groups/per opening and $cm^2$ top surface of framework is 80 to 750 nmol/per opening and $cm^2$.

In a preferred embodiment, the number of sulfate, sulfonate or sulfate and sulfonate groups/per opening and $cm^2$ top surface of framework is in particular 150 to 400 nmol/per opening and $cm^2$.

In a preferred embodiment, the number of sulfate, sulfonate or sulfate and sulfonate groups/per opening and $cm^2$ top surface of framework is in particular 450 to 700 nmol/per opening and $cm^2$.

The present invention relates in particular to a wound dressing comprising a polymeric flat framework having a coating of a polymer network, the polymer network comprising at least one polyionic polymer component covalently bonded to at least one of at least one uncharged polymer component and at least one non-polymeric cross-linking component thereby forming the polymer network, wherein the at least one polyionic polymer component comprises sulfate, sulfonate or sulfate and sulfonate groups, wherein the polymeric flat framework has a top and a bottom surface and is composed of a multitude of openings in the top surface of the framework and wherein the ratio of the area of the openings to the area of the surface containing no openings, each in the top surface of the framework, is from 0.5 to 6 and the number of sulfate, sulfonate or sulfate and sulfonate groups/total area of the surfaces of the framework is from 100 to 950 $nmol/cm^2$.

The present invention relates in particular to a wound dressing comprising a polymeric flat framework having a coating of a polymer network, the polymer network comprising at least one polyionic polymer component covalently bonded to at least one of at least one uncharged polymer component and at least one non-polymeric cross-linking component thereby forming the polymer network, wherein the at least one polyionic polymer component comprises sulfate, sulfonate or sulfate and sulfonate groups, wherein the polymeric flat framework has a top and a bottom surface and is composed of a multitude of openings in the top surface of the framework and wherein the ratio of the area of the openings to the area of the surface containing no openings, each in the top surface of the framework, is from 0.5 to 6 and the number of sulfate, sulfonate or sulfate and sulfonate groups/opening is 1 to 8 in nmol/opening.

In a preferred embodiment, the number of sulfate, sulfonate or sulfate and sulfonate groups/opening is in particular 2 to 4 in nmol/opening.,

In a preferred embodiment, the number of sulfate, sulfonate or sulfate and sulfonate groups/opening is in particular 5 to 8 in nmol/opening.

The present invention relates in particular to a wound dressing comprising a polymeric flat framework having a coating of a polymer network, the polymer network comprising at least one polyionic polymer component covalently bonded to at least one of at least one uncharged polymer component and at least one non-polymeric cross-linking

component thereby forming the polymer network, wherein the at least one polyionic polymer component comprises sulfate, sulfonate or sulfate and sulfonate groups, wherein the polymeric flat framework has a top and a bottom surface and is composed of a multitude of openings in the top surface of the framework and wherein the ratio of the area of the openings to the area of the surface containing no openings, each in the top surface of the framework, is from 0.5 to 6 and the number of sulfate, sulfonate or sulfate and sulfonate groups/per opening and $cm^2$ top surface of framework is 80 to 750 nmol /per opening and $cm^2$.

In a preferred embodiment, the number of sulfate, sulfonate or sulfate and sulfonate groups/per opening and $cm^2$ top surface of framework is in particular 150 to 400 nmol /per opening and cm2.

In a preferred embodiment, the number of sulfate, sulfonate or sulfate and sulfonate groups/per opening and $cm^2$ top surface of framework is in particular 450 to 700 nmol /per opening and cm2

The present invention also relates to a method for producing a wound dressing, in particular according to the present invention, comprising the following method steps:

a) providing at least one polymeric framework, at least one polyionic polymer, in particular poly-(4-styrenesulfonic acid-co-maleic acid), and at least one uncharged polymer, in particular a functionalized uncharged polymer, or/and at least one non-polymeric crosslinker molecule, and optionally further components, in particular a reaction medium,

b) mixing the uncharged polymer or non-polymeric crosslinker molecule with the polyionic polymer, in particular the poly-(4-styrenesulfonic acid-co-maleic acid), preferably in a reaction medium; and

c) applying the mixture obtained in step b) to the polymeric framework, thereby forming a polymer network of the at least one polyionic polymer, in particular the poly-(4-styrenesulfonic acid-co-maleic acid) component, and the at least one uncharged polymer component or/and a non-polymeric crosslinking component, so as to coat the polymeric framework and obtain the wound dressing.

Thus, in step a) there is provided at least one polymeric framework, a polyionic polymer, in particular poly-(4-styrenesulfonic acid-co-maleic acid), and at least one of at least one uncharged polymer, in particular a functionalized uncharged polymer, and at least one non-polymeric crosslinker molecule, which means that there is provided in addition to the at least one polymeric framework a polyionic polymer, in particular the poly-(4-styrenesulfonic acid-co-maleic acid), and at least one uncharged polymer, or a polyionic polymer, in particular the poly-(4-styrenesulfonic acid-co-maleic acid), and at least one non-polymeric crosslinking component or a polyionic polymer, in particular the poly-(4-styrenesulfonic acid-co-maleic acid), and at least one uncharged polymer and at least one non-polymeric crosslinking component.

In a preferred embodiment of the present invention, the reaction medium is water, preferably deionised water.

In a preferred embodiment of the present invention, the applying step c) is a spray-coating step.

In a preferred embodiment of the present invention, the applying step c) is an impregnating step.

In a preferred embodiment of the present invention, the applying step c) is a roll-to-roll step.

In a preferred embodiment of the present invention, the applying step c) is a casting step.

In a preferred embodiment, the, preferably functionalized, uncharged polymer has at least two functional groups, in particular end group, selected from the group consisting of amino group, thiol group, maleimide group, vinylsulfone group, acrylate group, carboxyl group and combinations thereof, wherein the at least two groups may be the same or different.

In a preferred embodiment, the, preferably functionalized, non-polymeric crosslinking molecule has at least two functional groups, in particular end group, selected from the group consisting of amino group, thiol group, maleimide group, vinylsulfone group, acrylate group, carboxyl group, hydroxylated aromatic group and combinations thereof, wherein the at least two groups may be the same or different.

Preferably provided in the at least one PIP, in particular PSS-MA, used in accordance with the invention and the at least one uncharged polymer and/or non-polymeric crosslinking molecule are at least two functional groups each selected from the group consisting of amino group, thiol group, maleimide group, vinylsulfone group, acrylate group, carboxyl group, and combinations thereof are each selected for the PIP, in particular the PSS-MA, and the uncharged polymer and/or non-polymeric crosslinking molecule such that the functional groups of the reactants can form a covalent bond with each other between the PIP, in particular PSS-MA, and the at least one uncharged polymer or between the at least one PIP, in particular PSS-MA, and the at least one non-polymeric crosslinking molecule to form a network.

The uncharged polymers are preferably selected from the group consisting of poly(ethylene glycols) (PEG), poly(2-oxazolines) (POX), polyvinylpyrrolidones (PVP), poly(vinyl alcohols) (PVA) and/or polyacrylamides (PAM), which in a preferred embodiment have at least two or more functional groups, in particular amino groups, cross linkable with the PIP-component, in particular PSS-MA, having carboxyl groups as a functional group.

The non-polymeric crosslinking molecules are preferably bifunctional crosslinking molecules which, in a preferred embodiment, have at least two or more functional groups, in particular amino groups, crosslinkable with the

PIP, in particular PSS-MA, having carboxyl groups as a functional group.

In a particularly preferred embodiment, in step iii) the carboxyl groups of PIP, in particular PSS-MA, or non-polymeric crosslinking molecules containing them are activated 1-ethyl-3-(3-dimethylaminopropyl) carbodiimide/N-hydroxysulfosuccinimide (EDC/sNHS).

In a preferred embodiment the present wound dressing is for use in a therapeutic method for treating wounds, in particular wounds of the skin or mucosa, in particular acute or chronic wounds, in particular diabetic wounds, in particular lower leg ulcers, foot ulcers and pressure ulcers.

In a preferred embodiment the present wound dressing is for use in a therapeutic method for treating wounds in the exudation, granulation or epithelialization phase.

In a preferred embodiment the present invention relates to a use for treating wounds in a subject in need thereof, in particular a human or animal subject. A subject in need thereof is in the context of the present invention also called a "patient".

In a preferred embodiment the present invention relates to a method for treating wounds in a subject in need thereof, in particular a human or animal subject, wherein a wound dressing of the present invention is applied, in particular fixed to a wound, in particular a wound of the skin or mucosa. In a preferred embodiment, the wound dressing is removed from the wound once the loading capacity of the PIP, in particular the PSS-MA, in respect to the chemokines removed from the wound has been reached.

In a preferred embodiment the present invention relates to a method for treating wounds in a subject in need thereof, in particular a human or animal subject, wherein a wound dressing of the present invention is applied, in particular fixed to a wound, in particular a wound of the skin or mucosa and a secondary wound dressing covering the wound dressing of the present invention is applied, preferably fixed, on the body-distal face of the present wound dressing.

The present wound dressing is preferably used after the wound has been cleaned and, if necessary, disinfected in a wound bed preparation procedure.

In the context of the present invention, the term "network" refers to a polymer network, comprising at least two polymer components as building units of said network, preferably at least two different polymer components, and wherein the polymer components are interconnected via chemical bonds with each other either directly or indirectly.

In the context of the present invention, the term "repeating unit" refers to a monomeric unit of a polymer. The term "at least one repeating unit" refers to the number of monomeric units making up the polymer and may be 1 to 100 000, preferably 10 to 10 000 or preferably 100 to 1 000 repeating units.

In the context of the present invention, the term "PIP component, the PSS-MA component or uncharged polymer component or non-polymeric crosslinking component" refers to the PIP unit, the PSS-MA unit, uncharged polymer unit and crosslinking unit being integrally present in the molecular network of the polymer network. The polymer network components are building units of the polymer network and provided to be used in the process of the polymer network preparation in form of PIP, PSS-MA, non-polymeric crosslinkers and uncharged polymers. The term "at least one component" refers to the number of building units making up the polymer network and may be 1 to 100 000, preferably 10 to 10 000 or preferably 100 to 1 000 building units.

The formula of the PSS-MA component showing the sulfonate (sulfonic acid) group and carboxylate (carboxylic acid) group is (with x and y each being an integer ≥1 of the repeating unit)

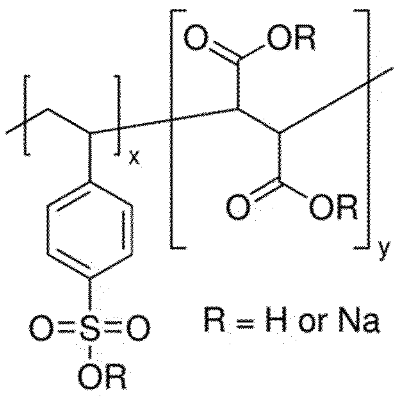

In the context of the present invention, the term "sulfonate group" refers to the anionic, deprotonated or to the protonated form of a sulfonic acid group, in particular to the anionic form.

In the context of the present invention, the term "sulfate group" refers to the anionic, deprotonated or to the protonated form of a sulfuric acid group, in particular to the anionic form.

In the context of the present invention, the term "chemokine" relates to cytokines or signaling proteins playing a

major role in biological processes, including morphogenesis and wound healing. In particular, pro-inflammatory chemokines are considered herein, such as IL-1, IL-8 (CXCL-8), MCP-1, TNF-alpha, LPS, CCL2, CCL3, CCL4, CCL5, CCL11 and CXCL10.

In the context of the present invention, the term "pharmaceutical substance" refers to a material, in particular compound, which exerts in a human or animal body a therapeutic, including prophylactic, action.

In the context of the present invention, the term "therapy" includes both a prophylactic and a curative treatment of an unhealthy state of a subject, in particular refers to a curative treatment.

In the context of the present invention, the term "use" being referred to in the context of a therapeutic method using the wound dressing means the offer, in particular, advertising, marketing, teaching, or prescription or sale or administration, in particular implantation, of the present wound dressing or a method using any one of it in a patient, such as evidenced for instance by prescription by a doctor, packing, packing leaflets, medical expert's information, expert's or doctor's or pharmacist's counselling, expert information in text or oral form, including in the internet, all of them also including off-label or cross-label use.

Where quantitative indications, in particular percentages, of components of a product or composition are given in the context of the present invention, these add up to 100% of the composition and/or product together with the other explicitly stated or expertly apparent further components of the composition or product, unless explicitly stated otherwise or expertly apparent.

In the context of the present invention, the term "a" or "the" is understood to refer to a single element or to a multitude of elements.

In the context of the present invention, the term "at least one" is understood to mean a quantity expressing a number of 1 or 2 or 3 or 4 or 5 or 6 or 7 or 8 or 9 or 10 and so on. In a particularly preferred embodiment, the term "at least one" may represent exactly the number 1. In another preferred embodiment, the term "at least one" may also mean 2 or 3 or 4 or 5 or 6 or 7.

In the context of the present invention, the term "composition" refers to a substance (in case a composition consisting of X is referred to) or to a mixture of substances (in case a composition comprising or containing or having or encompassing X is referred to).

If in context of the present invention a "presence", a "containing" or a "content" of a component is expressed, mentioned or implied in an amount of 0 [unit], in particular mg/kg, μg/kg or wt.%, this means that the respective components are not present in a measurable amount, in particular are not present.

The number of decimal places indicated corresponds to the precision of the measurement method used in each case.

If, in the context of the present invention, the first and second decimal places or the second decimal place are/is not indicated for a number, the latter is/are to be set as zero.

Where in the context of the present invention a "comprising", a "containing", an "encompassing", a "having" or a "content" of a component is expressly mentioned or implied, this means that the respective component is present, in particular present in a measurable amount.

In the context of the present invention, the terms "comprising", "encompassing" and "containing" are understood to mean that in addition to the elements explicitly covered by these terms, other elements not explicitly mentioned may be added. In the context of the present invention, it is also understood by these terms that only the explicitly mentioned elements are covered and that no further elements are present. In this particular embodiment, the meaning of the terms "comprising" and "containing" is synonymous with the term "consisting of", which term, that means "consisting of", means that the element referred to is the only element present, thereby excluding the presence of further elements. Furthermore, the terms "comprising", "encompassing" and "containing" also cover compositions which, in addition to the explicitly mentioned elements, also contain further elements which are not mentioned but which are of a functional and qualitatively subordinate nature. In this embodiment, the terms "comprising" and "containing" are synonymous with the term "consisting essentially of".

In the context of the present invention, the term "and/or" is understood to mean that all members of a group connected by the term "and/or" are represented both cumulatively with respect to each other in any combination, and alternatively with respect to each other. Exemplarily, for the expression "A, B and/or C" relating to members A, B and C of a group, the following disclosure is to be understood thereunder: i) (A or B or C), or ii) (A and B), or iii) (A and C), or iv) (B and C), or v) (A and B and C), or vi) (A and B or C), or vii) (A or B and C), or viii) (A and C or B). Thus, the term "and/or" is understood to refer to a cumulative combination of all members of the group, to any combinations of at least two and more members of said group and to each single member of the group. Thus, the term "and/or" is understood to be equivalent to the term "A or B or a combination thereof.

In the context of the present invention, individual components or constituents of a composition or of one of its components, determined quantitatively in relative form, in particular in percentages, preferably add up to 100% by weight of the respective composition referred to or of the composition or, if referred to, of a component thereof, unless otherwise stated.

In the context of the present invention, the term "framework" is meant to be the structural body of a wound dressing, that means the component of a wound dressing making up the geometry, form and physical structure of the wound dressing. A framework can also be termed to be a support or a carrier.

In the context of the present invention, the term "flat framework" refers to a framework with a defined three-dimensional form, wherein the x- and y-components of a cartesian coordinate system defining the three space dimensions of the form are defining the length and width of the framework, while the z-component defines the thickness of the framework and wherein the x- and y-components each are at least 2 times, preferably at least 3, at least 4, at least 5, at least 6, at least 7, at least 8, at least 9, at least 10, preferably at least 50 times greater than the z-component. The x- and y-components together are defining the top and bottom surface of the framework, which is preferably a flat or even surface. The z-component together with either the x- or the y-component is defining the side surface of the side walls of the framework. Preferably, the top and bottom surfaces are parallel to each other. Preferably, the openings of the framework extend from the top surface to the bottom surface over the entire thickness of the framework, preferably without altering their form, their cross-section and outline, in particular shape, in x- and y-dimensional direction, and their surface area. The openings are surrounded over their entire thickness by side walls of frames. Preferably, the structure, in particular form, area and outline of openings and of frames, of the top surface is identical to that of the bottom surface.

The framework of the wound dressing is comprised, in particular made up, of multiple openings, in particular at least two openings, which are located in the top surface in the framework and extend through the framework to the bottom surface. The parts of the framework which are surrounding and defining the openings in their shape and size are termed frames, a frame is thus a part of the framework. Each opening thus has a corresponding frame. The framework of the wound dressing is thus comprised, in particular made up, of multiple openings, in particular at least two openings and a multitude of frames, in particular at least two frames, wherein each frame is, at least partially, preferably completely, surrounding and thereby defining the outline (also termed circumference) of a corresponding opening. Thus, the frame forms the side walls of an opening. In case the frame is at an edge of the framework it also forms the side wall of the framework. Thus, the frame has at least a top surface, a bottom surface and surfaces of the side wall of the frame, in particular which face the inside of the opening and possibly, depending upon location and form of the frame, form an outer surface of the side wall, such as at an edge of the framework, The frames are preferably integral elements of the framework and in their entirety build up the framework.

In the context of the present invention, the term "area of frame surfaces in the top surface of the framework" refers to the sum of the surfaces of all frames located in the top surface of the framework. It is thus defined as the solid framework area in the top surface plane of the framework, that means it is therefore the transport limiting surface area. Said area is thus the surface area containing no openings and is thus the complete solid or closed surface in the top surface of the framework. The "area of frame surfaces in the top surface of the framework" represents the difference between the complete surface of the top surface of the framework and the area of all openings in the top surface of the framework

In the context of the present invention the term "area of openings in the top surface of the framework" is defined as the open space area in the top surface of the framework. Said area is the sum of the areas of all openings in the top surface of the framework.

The "ratio of the area of the openings to the area of the frame surfaces, each in the top surface of the framework" thus is the "ratio of the area of the openings to the surface area containing no openings, each in the top surface of the framework" (ratio of open space area to closed space area in the top surface of the framework). This ratio describes the structure of the framework and thus the permittivity for fluid transport across the framework that means a ratio of 1 means 50% of the total framework top surface is open and 50% of the total framework top surface is the closed framework surface formed by the top surfaces of the frames.

In the context of the present invention, the term "total area of frame surfaces" refers to the area of all surfaces of all frames in a framework, in particular all surfaces of the frames, that means preferably the top and bottom surface of the frames and the surfaces of the frames, which form the side walls of the opening and the side walls of the framework, in particular over the entire thickness of the framework.

The "total area of frame surfaces" that means the area of all surfaces of all frames of a framework, is the same as the "total area of the surfaces of the framework".

The "number of sulfate/sulfonate groups per total area of frame surfaces (nmol/$cm^2$)" is also referred to as "the number of sulfate/sulfonate groups per total area of the surfaces of the framework (nmol/$cm^2$)".

The "number of sulfate/sulfonate groups per total area of frame surfaces (nmol/$cm^2$)" is the "loading density of the sulfate/sulfonate groups per total area of the surfaces of the framework".

The sizes, dimensions and the areas of the surfaces and openings of the framework and the single frames making up the framework can be determined with microscopy techniques such as light microscopy (LM) or scanning electron microscopy (SEM) using common image processing tools. Based on the microscopic characterization of the geometric dimensions the ratio of the area of the openings to the area of the frame surfaces, each in the top surface of the framework, can be calculated by dividing the total area of all openings in the top surface of the framework by the total area of all frames in the top surface of the framework (closed area in the top surface, i.e. framework surface) of any given total

framework surface (Ratio of the area of the openings to the area of the frame surfaces = total area of all openings in the top surface of the framework/total area of all frames in the top surface of the framework). Based on the microscopic characterization of the geometric dimensions also the number of sulfate, sulfonate or sulfate and sulfonate groups per total area of frame surfaces and per total area of the surfaces of the framework can be determined.

The terms chemokine "binding", "scavenging" and "sequestration" are used as synonyms, i.e. it means binding of chemokines by the polymer coating/wound dressing and therefore the removal of these pro-inflammatory mediators from the wound exudate/from wound fluid.

"Wound exudate" or "wound fluid" or "wound secretion" are used as synonyms.

The term "multitude" refers to a number of at least two, preferably at least 100, preferably at least 500, preferably at least 1000, preferably at least 5000 and preferably at least 6000,

Further preferred embodiments of the present invention are apparent from the dependent claims.

The present invention will be explained in more detail in the following examples and accompanying figures which are not to be understood as limiting.

The figures show:

Figure 1 a schematic view of a framework (left) and a single frame thereof (right) of a wound dressing of the invention, not drawn to scale, shown from the top view and

Figure 2 a schematic view of a single frame displaying the x-, y- and z-dimensional extensions of one opening/frame of a wound dressing of the invention, not drawn to scale

Reference signs:

| | |
|---|---|
| 1 | Opening area, |
| 2: | area of frame surface in the top surface of the framework (closed area), |
| 3 | width of the opening; |
| 4 | length of the opening |
| 5: | width of the frame (x-dimension), |
| 6 | length of the frame (y-dimension), |
| 7 | thickness of the frame that is identical with the thickness of the opening (z-dimension), |
| 8 | inner surfaces of the frame on the inner side walls of the opening. |
| 50 | opening |
| 60 | frame |
| 70 | side wall of framework |
| 100 | framework |

**Example 1: Coating of fibre meshes with polyionic polymer networks**

The preparation of the different wound dressings was carried out by coating the fibre mesh framework material with the polymer network containing one polyionic polymer (PIP) crosslinked with four-arm amine-terminated PEG (4-arm PEG, (MW 10 000 g/mol, Jenkem technology, USA). The polyionic polymers was poly(4-styrenesulfonic acid-co- maleic acid) with the molar ratio 4-styrenesulfonic acid:maleic acid 1:1, MW 20 000 g/mol, manufacturer no.: 434558, Sigma-Aldrich, Germany, (PSS1M). The polyionic polymer and the four-arm amine-terminated PEG, were used to form covalently crosslinked polymer networks around the fibre mesh by the aid of carbodiimide chemistry. The composition for the polymer coating is given in Table 1. The framework material was a polyester knitted fabric with the following characteristics of a 7.5 x 10 cm dressing (top surface area of the framework/dressing: 75cm$^2$): The framework material is a textile in the form of a fibre mesh that forms a framework with a number of 6199 +/-5% openings (83+/-5% /cm$^2$) in its top surface which openings are surrounded by multifilament polyester textile frames in form of fibres to form openings with the following dimension (opening is open area not covered by the frame): width (opening) 0.86 mm, length (opening): 0.89 mm, thickness 0.18 mm (all values +/- 5%), total area of one frame unit in the top surface (frame unit: open area plus textile frame area, each in the top surface of the framework) 1.21$\pm$5% mm$^2$, area of one opening in the top surface of the framework 0.77$\pm$5% mm$^2$, area of one frame surface of one opening in the top surface of the framework (area covered by textile frame struts/fibres at the top surface of the framework): 0.44$\pm$5% mm$^2$. Furthermore, the total area of the surfaces of one single frame, which includes the top surface, the bottom surface and the surfaces perpendicular to the framework defining the outline of the opening and defining the outline of the framework (each calculated from the frame thickness perpendicular to the framework top surface) have been measured to be 1.14$\pm$5% mm$^2$ (framework 1, Texinov medical textiles, France). See

Figure 1 and 2.

Figures 1 and 2 show the top and bottom surfaces of one flat polymeric framework, wherein the polymeric flat framework characterised by a particular length, width and thickness of the total framework and of each frame unit has a top and a bottom surface and is composed of a multitude of openings and a multitude of frames surrounding the openings.

Figure 1 shows the total area of the frame (60) in form of a fibre of a framework (100), the area (1) of a single opening (50) and the area (2) of a frame (60) surface surrounding the opening (50), each in the top surface of the framework (100). Indicated are also the width (3) of the opening (50) and the length (4) of the opening (50).

Figure 2 shows the total area of the surfaces of one frame (60), which are defined in their x-(width, 5) and y-( length, 6) dimensional extension (width times length) and the surfaces of the frame (60) extending in z-dimensional direction (thickness, 7) forming the side walls of the opening (50), in particular forming the outline of the opening (50) and, in case the frame (60) is at an edge of the framework (100), forming side walls (70) of the frame (60) with an outer surface and thus of the framework (100). The thickness of the frame (60) and framework (100) is identical to the thickness of the opening (50). The total area of the surfaces of a single frame (60) is the sum of the area of all surfaces of the single frame (60), including surfaces (8) of the side wall (70) facing the inside of the opening (50) and outer surfaces of the frame (60). The "total area of the frame surfaces" is the sum of the total areas of the surfaces of all frames (60) of a framework (100).

To manufacture the wound dressings (Type 1-4), the polymer network building units of the polymer network coating material was first dissolved at the following concentrations (details in Table 1). For that purpose, the polyionic polymer was dissolved in saline 0.9% isotonic solution (B. Braun Ecoflac Plus) at 4°C for 30 seconds at 500 rpm using a vortex mixer (IKA, Germany) (weights and volumes according to Table 1). In addition, 4-arm PEG, 1-ethyl-3-(3-dimethylaminopropyl)carbodiimide (EDC; Sigma-Aldrich, Germany) and N-hydroxysulfosuccinimide (sulfo-NHS; Sigma-Aldrich, Germany) were dissolved in the same manner. To ensure complete dissolution of the polymer network building units, these solutions were additionally treated for 5 min at 4°C with an ultrasonic bath (RK 100H, Bandelin, Germany).

*Table 1 Preparation parameters for 1 ml of the reaction mixture of the polymer network for the different wound dressing types, with respect to the weight of the polymers and carbodiimide activators and the volume of the saline as solvent.*

| Woun d dressi ng types | PIP | PIP MW [kD a] | PIP weigh t [mg] | 4-arm PEG weigh t [mg] | EDC weigh t [mg] | NHS weigh t [mg] | EDC volu me [μl] | NHS volu me [μl] | PIP volu me [μl] | 4-arm PEG volu me [μl] |
|---|---|---|---|---|---|---|---|---|---|---|
| **Type 1-4** | PSS1 M | 20 | 30.0 | 30.0 | 5.0 | 2.5 | 45 | 25 | 595 | 335 |

To produce the wound dressing of type 1, type 2, type 3 and type 4, a spray coating machine was used. The PIP and sulfo-NHS solutions (with the composition indicated in Tabe 1, total volumes to be adjusted for the respective coating process) were filled into a 30 ml syringes while 4-arm PEG and EDC were filled in another 30 mL syringe (BD Plastipak with Luer 30 ml, manufacturer no.: 301229 BD, Germany and BD Blunt fill needle 18G, manufacturer no.: 305181, BD, Germany). The polymer precursors and crosslinkers were afterwards mixed and pumped using syringe pump (PHD Ultra, Harvard Apparatus, USA) and sprayed using a commercial spray head (atomizing nozzle model SF8010SS, ExAir cooperation, USA) onto the polyester textile framework. The polymer mixture was sprayed for different times on the area of a 7.5 cm x 10 cm polyester textile framework in order to reach the coating amounts in mg coating/dressing for types 1, 2, 3, 4 as displayed in Table 2.

After spray coating the polymerization of the polymer network coating for all dressing types took place for at least 8-12 hours at 4°C where the EDC/NHS activated carboxylic acid groups of the PIPs react with the amine groups of the 4-arm PEG to form amide groups as stable, covalent crosslinks. Upon polymerization, the polymer network is formed surrounding the single frames of the framework allowing for a stable coating.

The dressings of type 1 - 4 (7.5 x 10 cm samples) were characterized prior and after the coating procedure by weighing the dry coating amount (after air drying of the dressings for 24 hours at 4°C) using a high precision micro balance (Sartorius, model: MC 210). The results are displayed in Table 2.

To determine the amount of PIP per dressing (which is also per framework) (mmol), the PIP weight % was estimated to be 39% of the dry weight of the polymer coating, according to Table 1:

Equation 1:

$$no.\,of\ moles\ PIP\ per\ dressing(mmol) = \frac{coating\ amount\ per\ dressing(mg) * \%\ dry\ PIP\ in\ coating\ mixture}{Mwt\ PIP\ (\frac{g}{mol})}$$

To calculate the no. (no: number) of sulfonates groups per dressing/framework, where one mol of the PIP poly(4-styrenesulfonic acid-co-maleic acid) contains 75 mol sulfonate groups,

Equation 2:

$$no.\,of\ moles\ of\ sulfate/sulfonate\ groups\ per\ dressing\ (\mu mol) = moles\ PIP\ per\ dressing\ (\mu mol) * sulfonate\ moles\ per\ PIP$$

To calculate the no. of sulfate/sulfonate groups per opening, where the no. of openings per framework/dressing top surface was 6199:

Equation 3:

$$\text{no. of mols sulfate/sulfonate groups per opening (nmol)} = \frac{\text{coating amount in one dressing (nmol)}}{no.of\ openings\ on\ dressing\ top\ surface}$$

(if openings with different size are present in the framework the total frame area have to be calculated accordingly).

To calculate the no. of sulfate/sulfonate groups per opening per $cm^2$ of the dressing/framework top surface, where the no. of openings per $cm^2$ dressing/framework top surface is 83 per $cm^2$:

Equation 4:

$$\text{no. of mols sulfate/sulfonate groups per opening per cm}^2\text{ dressing top surface }\left(\frac{\text{nmol}}{\text{cm}^2}\right) = \text{no. of mols sulfate/sulfonate groups per opening (nmol)} * \text{no. of openings per cm}^2\text{ dressing top surface}$$

The total area of the frame surfaces which are covered by the polymer coating, that means preferably the top surface, bottom surface and the surface of the frame surrounding each opening and, if located at an edge of the framework, defining the outline of the framework, was calculated based on the measurement of geometric dimensions of the dressing framework from SEM images with manufacturer image analysing software (Quattro ESEM, Thermo Fischer Scientific, Germany) showing the frame dimensions resulting in total frame surface area = 1.14±5% in $mm^2$. This was used to calculate the no. of sulfate or sulfonate groups per total area of the surfaces of one frame (from all sides) as follows:

Equation 5:

$$\text{no. of sulfate or sulfonate groups per total surfaces area of one frame } \left(\frac{\text{nmol}}{\text{cm}^2}\right) = \frac{\text{no. of mols sulfate/sulfonate groups per opening (nmol)}}{total\ area\ of\ all\ surfaces\ of\ one\ frame\ (cm2)}$$

The number of sulfate and/or sulfonate groups per total area of all surfaces of all frames (which is the "total area of frame surfaces") is then calculated by equation 6.

Equation 6:

$$\text{no. of sulfate or sulfonate groups per total area of frame surfaces } \left(\frac{\text{nmol}}{\text{cm}^2}\right) = \text{no. of sulfate or sulfonate groups per total surfaces area of one frame } \left(\frac{\text{nmol}}{\text{cm}^2}\right) x \frac{no.of\ openings\ per\ framework}{no.of\ frames\ per\ framework}$$

**Example 2: Exudate transport**

The coated wound dressings were swollen in phosphate-buffered saline for at least 3 hours prior to further characterization of the coated dressing materials.

Wound exudate is a complex fluid produced during the healing process, particularly during the inflammatory phase. Its composition includes water, electrolytes, proteins (e.g., albumin, globulins, fibrinogen, enzymes, inflammatory cytokines and chemokines) and cellular components such as white blood and inflammatory cells (*doi.org/10.12968/bjcn.2003.8.sup3.11577*). The specific composition can vary depending on the wound type, healing stage, and presence of infection or inflammation (*doi.org/10.1111/iwj.13960*). Elevated levels of proteins, particularly plasma proteins like fibrinogen, can significantly increase exudate viscosity *(doi.org/10.1016/j.ijpx.2024.100288).* These proteins can form gel-like structures that thicken the fluid. Accordingly, it is important to mimic these significantly differing wound exudate viscosities by testing different artificial wound exudate that mimics these different exudate states. Therefore, the exudate transport through the wound dressing have been analyzed. Consequently, two solutions with different PEG8000 (manufacturer no.: 0159, VWR, Germany) concentrations that have been mixed with human serum solution were fabricated in order to mimic wound exudate with high and low viscosities. The two solutions were allowed to pass vertically through the different dressings under the influence of gravity. For that purpose, the PEG8000 powder was completely dissolved in deionized, ultrapure water with concentrations of 50% or of 10% w/v corresponding to a viscosity of 325 and 8.9 mPa.s respectively. The final solutions contained 80% PEG800 solutions and 20% human serum solution, extracted from the blood of healthy donors, to simulate high and low viscosities of wound exudate. 1mL of the respective solutions were pipetted on a horizontally stretched wound dressing and the weight of the solution that could vertically pass through the mesh after 1 minute, under the effect of gravity was measured by a high precision micro balance (Sartorius, model: MC 210 and recorded in Table 3 as % solution initially added.

Type 1 and Type 2 wound dressing did clearly outperform the Type 4 with effective exudate transport independent of the viscosity of the wound exudate-mimicking solution. Type 3 dressing displayed reduction in the exudate transport while Type 4 allowed very little or no exudate to pass through the dressing at both high and low viscosities (results see Table 2).

**Example 3: Determination of the chemokine-binding properties**

The sequestration of pro-inflammatory chemokines is crucial to stop excessive and chronic inflammatory processes and thus to enable healing of chronic wounds, as it has been shown by Lohmann et al. (*doi.org/10.1126/scitranslmed.aai9044*) and Schirmer et al. (*doi.org/10.1002/advs.202100293*). In particular, up to 500-fold elevated levels of the chemokine IL-8 have been found in wound exudates of chronic venous ulcer patients that fuel excessive and chronic inflammatory processes and thus IL-8 seems to be one of the most important pro-inflammatory mediators found in human chronic wound patients (*doi.org/10.1016%2Fj.jvs.2008.11.049*). Therefore, as the most important performance indicator the chemokine scavenging performance of the wound dressings was analyzed by exposing the wound dressing to chemokine containing simulated wound fluid (SWF), a solution that closely mimics the salinity and albumin concentration

of human wound exudate (*doi.org/10.1046/j.1524-475x. 1999.00442.x* and *doi.org/10.1021/pr100456d)* . For that purpose, the chemokine IL-8 was added at a concentration of 200 ng/mL to SWF prepared by dissolving 5% bovine serum albumin (manufacturer no.: A7030, Sigma-Aldrich, Deutschland), 142 mM sodium chloride (Sigma-Aldrich, Deutschland), 2.5 mM calcium chloride (Sigma-Aldrich, Deutschland) and 0.05% Proclin (Sigma-Aldrich, Deutschland) in deionized, ultrapure water, on a plate shaker (VWR, Deutschland) at 300 rpm for one hour and afterwards the SWF was then sterile filtered using a 0.2 μm syringe filter.

Subsequently, 1 $cm^2$ wound dressing samples (obtained by punching respective sample sizes out of the 7.5 x 10 cm wound dressing samples using a round puncher) were incubated in 1 mL incubation solution containing the IL-8 in SWF at a concentration of 200 ng/mL in a 24-well plate for 24 hours at room temperature on a plate shaker (VWR, Deutschland) at 100 rpm. Afterwards, the chemokines containing SWF was collected and quantified by ELISA technique according to manufacturer's protocol, to determine the amount of unbound protein Human IL-8 (ELISA Kit, Catalog # 88-8086-77, Thermofischer, Germany). The amount of unbound protein was then subtracted from the amount of the unbound IL-8 after incubation with the uncoated polyester textile sample (negative control), also determined by ELISA, to identify the amount of bound protein for each dressing type.

Surprisingly, the dressings of type 2 and type 3 were able to effectively bind 96% the IL-8 from the SWF, while type 1 resulted in considerable reduction in binding of the IL-8 to 76%, indicating a significant reduction in IL-8 scavenging capacity (results see Table 2). Moreover, type 4 dressing showed a slightly reduced binding of 93% (results see Table 2).

In Table 2 the results of the polymer coating amount, the exudate transport and IL-8 scavenging for the 4 types of wound dressing are summarized.

Accordingly, there is an unexpected high value of exudate transport of high viscosity for type 1 and type 2 and type 3 dressing of 100%, 98% and 81 % as well as for the exudate transport of low viscosity of 97%, 95% and 76%, while for dressing type 4 the exudate transport drops dramatically to 11% and 5% respectively. Rapid transport of exudate through the dressing is essential for the removal of contaminants, cell debris, excess proteins, enzymes, and water from the wound area, thereby initiating the healing process. The dressing must facilitate the swift movement of both highly viscous exudate, common during the inflammatory phase, and less viscous exudate at subsequent stages. This balance promotes healing and prevents maceration of the surrounding tissue. Type 2 and Type 3 wound dressing exhibits this advantageous characteristic.

Table 2: Summarized results of wound dressings of type 1 - type 4: first row: polymer coating amount per dressing top surface (which is also per framework top surface) [mg/75 $cm^2$] n>3, value ±12%, second row: no. of sulfonate or sulfate groups/opening (nmol) n>3, value ±12%, third row: no. of sulfonate or sulfate groups/opening of 1 $cm^2$ dressing top surface are (nmol/$cm^2$) n>3, value ±12%, fourth row: no. of sulfate or/and sulfonate groups/total area of frame surfaces of the framework, including top, bottom and inner surfaces (nmol/$cm^2$) n>3, value ±12%, $5^{th}$ row: Ratio of area of the openings (open space) to the area of the frame surfaces (closed space) area (each in the top surface of the framework) value ±5%,, $6^{th}$ and $7^{th}$ row: Measurement of exudate transport; the % of high (HV) and low viscous (LV) artificial wound exudate solution to vertically pass through the wound dressing under the effect of gravity (Mean ± %-error, n = 4), $8^{th}$ row: % of IL-8 bound after incubation of the wound dressing in simulated wound fluid containing IL-8 for 24 hours. (Mean ± %-error, n =18)

| | **Type 1 (control)** | **Type 2 (invention)** | **Type 3 (invention)** | **Type 4 (control)** |
|---|---|---|---|---|
| | Mean | Mean | Mean | Mean |
| Coating amount /dressing top surface[mg/ 75 $cm^2$] | 2.9 | 12.8 | 28.3 | 54.8 |
| no. of sulfonate or sulfate/open ing (nmol) | 0.69 | 3.02 | 6.67 | 12.93 |
| no. of sulfonate or sulfate/open ing of 1$cm^2$ top surface of fra-mework (nmol/$cm^2$) | 56.8 | 249.6 | 551.1 | 1069.0 |
| no. of sulfate or sulfonate /total area of frame surfaces (nmol/$cm^2$) | 60.1 | 263.9 | 582.7 | 1130.3 |
| Ratio of the area of the openings to the area of the frame surfaces | 1.74 | 1.74 | 1.74 | 1.74 |
| Exudate transport (HV) | 100±3% | 98±4% | 81±7% | 11±9% |

(continued)

| | Type 1 (control) | Type 2 (invention) | Type 3 (invention) | Type 4 (control) |
|---|---|---|---|---|
| | Mean | Mean | Mean | Mean |
| Exudate transport (LV) | 97±3% | 95±3% | 76±4% | 5±5% |
| IL-8 scavenging [%] | 76±12% | 96±3% | 96±1% | 93±4% |

Interestingly, in parallel type 2 and type 3 dressing demonstrated an unexpectedly high IL-8 scavenging value of 96%, while type 1 dressing significantly dropped to 76%. This suggests that type 2 and type 3 dressing not only promotes high exudate transport but also exhibits a high IL-8 binding capacity, which is anticipated to enhance wound healing performance (see *doi.org/10.1126/scitranslmed.aai9044* and *doi.org/10.1002/advs.202100293).*

Without being limited to any particular theory or mechanism of operation the number of sulfate or/and sulfonate groups/opening (nmol), the no. of sulfonate or/and sulfate/opening of per $cm^2$ dressing top surface ($nmol/cm^2$) as well as the no. of sulfate or sulfonate/total area of the frame surfaces ($nmol/cm^2$) seems to be crucial for both application related properties of the wound dressings: Exudate transport and inflammatory chemokine binding. Both processes are obviously influenced by the number of under physiological conditions negatively charged sulfate and/or sulfonate groups per opening or per frame surface area that may form strong electrostatic interactions with positively charged surface groups of proteins (protonated side chains of lysin and arginine amino acids). Accordingly, pro-inflammatory chemokines such as IL-8 and MCP-1 will be bound *(doi. org/l 0.1126/scitranslmed aai9044* and *doi.org/10.1002/advs.202100293)* as well as strong interactions may also occur with other large wound exudate proteins such as fibrinogen, albumin, as well as cell debris (*doi.org/10.1111/iwj.13960*). Accordingly, a high sulfate or sulfonate group number per opening or per frame surface area may facilitate binding of chemokines as well as binding and even crosslinking of significantly larger wound exudate proteins such as fibrinogen, albumin and collagens and accordingly it may decrease the exudate transport. It has been reported that charged large structural proteins such as fibrinogen, collagens, fibronectin and cell debris may form highly viscous wound exudates (*doi.org10.1111/iwj.13960*). On the other hand, the permittivity for wound exudate may be dramatically influenced by the ratio of the area of the openings in the top surface of the framework to the area of the frames in the top surface of the framework, in here a large ratio (i.e. a large area of openings) may facilitate wound exudate transport. Furthermore, small openings coated with the polymer coating may improve exudate transport due to capillary forces, whereas in opposite especially with high viscous exudate small openings may block and therefore prevent exudate transport. Finally, high blocking of exudate transport may also interfere with high IL-8 binding due to the formation of a barrier that prevents diffusion of the chemokines to the dressing surface.

Surprisingly, intermediate values of 3-7 nmol sulfate or sulfonate groups/opening, intermediate values sulfonate or sulfate and sulfonate groups/total area of frame surfaces ranging from 100 - 950 $nmol/cm^2$, and intermediate values of the number of sulfate, sulfonate or sulfate and sulfonate groups/per opening and $cm^2$ top surface of framework from 80 to 750, as well as a 1.74 ratio of area of the openings to the area of the frames (type 2 and type 3 dressing properties) promote both optimal high IL-8 binding as well as allowing for optimal exudate transport, whereas values for the number of sulfate or sulfonate groups/opening above 12 nmol sulfate or sulfonate groups/opening did almost completely prevent exudate transport and slightly reduce chemokine binding. Similarly, values of 0.5-6 of ratio of opening's area to frame structure area (corresponding to 33 to 85% of opening area) seems beneficial to promote exudate transport at reasonable high scavenging of IL-8. Therefore, in Table 3 other suitable framework materials have been analyzed for their ratio of the area of the openings to the area of the frames, each in the top surface of the framework.

Table 3: Characterization of the ratio of the area of the openings to the area of the frames, each in the top surface of the framework, based on SEM measurements of different suitable frameworks, (Framework 1, Texinov, France; Texinov 601, Texinov, France; URGO Start, Urgo, France; Tegaderm, 3M, USA; PU-foam: Polyurethane foam, Mölnlycke, Sweden)

| | Framework 1 | | Texinov 601 | | URGO Start | | Tegaderm | | PU-foam | |
|---|---|---|---|---|---|---|---|---|---|---|
| | Mean | SD | Mean | SD | Mean | SD | Mean | SD | Mean | SD |
| Ratio of area of openings to area of frames | 1.74 | 0.09 | 3 | 0.3 | 1.52 | 0.32 | 0.73 | 0.05 | 6 | 0.5 |

They can be similarly coated applying the procedure and polymer coating described in example 1 (data not shown).

Accordingly, the two parameter groups referring to the number of negatively charged sulfate, sulfonate or sulfate and sulfonate groups per opening, or per total area of frame surfaces, or per opening and $cm^2$ top surface of framework on the one hand as well as the ratio of area of the openings to the area of the frames on the other hand influence chemokine binding and exudate transport allow for both: high chemokine binding and high exudate transport.

## Claims

1. A wound dressing comprising a polymeric flat framework having a coating of a polymer network, the polymer network comprising at least one polyionic polymer component covalently bonded to at least one of at least one uncharged polymer component and at least one non-polymeric cross-linking component thereby forming the polymer network, wherein the at least one polyionic polymer component comprises sulfate, sulfonate or sulfate and sulfonate groups, wherein the polymeric flat framework has a top and a bottom surface and is composed of a multitude of openings and a multitude of frames surrounding the openings and wherein the ratio of the area of the openings to the area of the frame surfaces, each in the top surface of the framework, is from 0.5 to 6 and the number of sulfate, sulfonate or sulfate and sulfonate groups/total area of frame surfaces is from 100 to 950 $nmol/cm^2$.

2. The wound dressing of claim 1, wherein the polyionic polymer component is a component selected from the group consisting of poly-(4-styrenesulfonic acid-co-maleic acid), poly(acrylic acid-co-2-acrylamido methylsulfonate), poly(acrylic acid-co-2-acrylamido ethanesulfonate), poly(acrylic acid-co-2-acrylamido ethanesulfate), poly(acrylic acid-co-4-(acrylamidomethyl)benzenesulfonate), poly(methyl methacrylate)-styrene sulfonate terpolymers, poly (sulfonated styrene-co-methyl methacrylate), poly (sulfonated styrene-co-acrylic acid-co-methyl methacrylate), sulfated poly(vinyl alcohol-co-acrylic acid), sulfonated poly(glutamic acid) and combinations thereof.

3. The wound dressing of claim 1 or 2, wherein the polymer network is a synthetic polymer network.

4. The wound dressing of any one of the preceding claims, wherein the at least one uncharged polymer component is selected from the group consisting of poly(ethylene glycol) (PEG) component, poly (2-oxazoline) (POX) component, polyvinylpyrrolidone (PVP) component, poly(vinyl alcohol) (PVA) component, polyacrylamide (PAM) component, and combinations thereof.

5. The wound dressing according to any one of the preceding claims, wherein the at least one uncharged polymer component is a poly(ethylene glycol) component, in particular a linear or multi-arm, in particular four- or eight-arm, poly(ethylene glycol) component.

6. The wound dressing of any one of the preceding claims, wherein the at least one non-polymeric cross-linking component is selected from the group consisting of ethylenediamine, propylenediamine (1,3-diaminopropane), butane-1,4-diamine, pentane-1,5-diamine (cadaverine), hexamethylene-1,6-diamine oxalic acid, malonic acid, succinic acid, glutaric acid, adipic acid, pimelic acid, N-(2-aminoethyl) maleimide, enzymatically cleavable peptides, and combinations thereof.

7. The wound dressing according to any one of the preceding claims, wherein the polymeric framework is a mesh, a foam, in particular a polymeric foam, a perforated plate, a perforated membrane or a perforated film.

8. The wound dressing according to any one of the preceding claims, wherein the polymeric framework is made of a synthetic polymer, in particular polyester, polyamide, cellulose, polyurethane, silicone polymer or polyethylene.

9. The wound dressing according to any one of the preceding claims, wherein the wound dressing comprises a swellable polymer, in particular CMC, pectin or gelatin.

10. The wound dressing according to any one of the preceding claims, wherein the smallest diameter of one opening is 500 $\mu m$ to 2 mm.

11. The wound dressing according to any of the preceding claims, wherein the wound dressing, in particular the coating, is non-adhesive.

12. The wound dressing according to any one of the preceding claims, wherein the wound dressing comprises at least one anti-microbial agent.

**13.** The wound dressing according to any one of the preceding claims for use in a therapeutic method for treating wounds, in particular wounds of the skin or mucosa, in particular acute or chronic wounds, in particular diabetic wounds, in particular lower leg ulcers, foot ulcers and pressure ulcers.

**14.** The wound dressing according to any one of the preceding claims for use in a therapeutic method for treating wounds in the exudation, granulation or epithelialization phase.

**15.** A method for producing a wound dressing, in particular according to any one of claims 1 to 14, comprising the following method steps:

a) providing at least one polymeric framework, at least one polyionic component, in particular poly-(4-styrenesulfonic acid-co-maleic acid), and at least one uncharged polymer, in particular a functionalized uncharged polymer, or/and at least one non-polymeric crosslinker molecule, and optionally further components, in particular a reaction medium,
b) mixing the uncharged polymer or non-polymeric crosslinker molecule with the polyionic component, in particular the poly-(4-styrenesulfonic acid-co-maleic acid), preferably in a reaction medium; and
c) applying the mixture obtained in step b) to the polymeric framework, thereby forming a polymer network of at least the polyionic component, in particular the least one poly-(4-styrenesulfonic acid-co-maleic acid) component, and the at least one uncharged polymer component or/and a non-polymeric crosslinking component, so as to coat the polymeric framework and obtain the wound dressing.

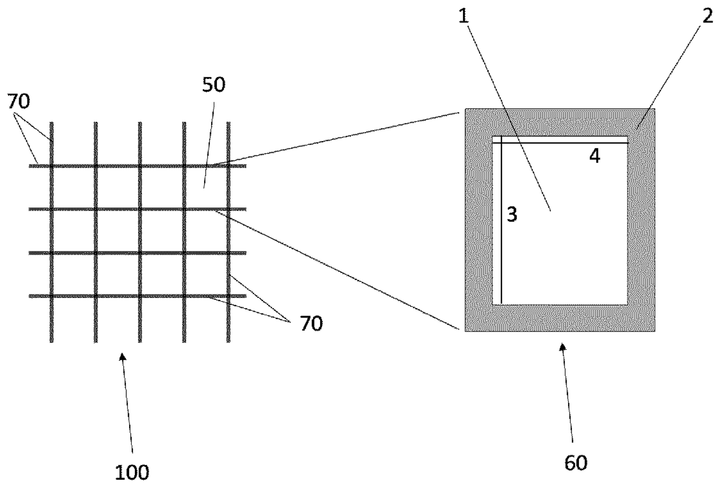


Figure 1

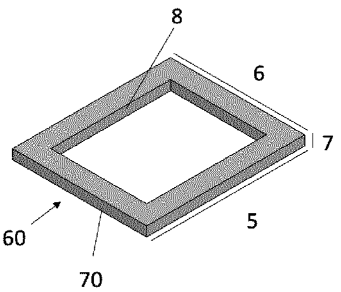


Figure 2

Europäisches Patentamt
European Patent Office
Office européen des brevets

# EUROPEAN SEARCH REPORT

Application Number
EP 24 22 2210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 4 487 876 A1 (LEIBNIZ INST POLYMERFORSCHUNG DRESDEN EV [DE]) 8 January 2025 (2025-01-08) * claims 1-15 * * example 2 * ----- | 1-15 | INV. A61L15/24 A61L15/26 A61L15/42 |
| A | US 2020/040147 A1 (SCHIRMER LUCAS [DE] ET AL) 6 February 2020 (2020-02-06) * claims 56-58, 71 * * paragraphs [0012], [0050] * ----- | 1-15 | |
| A | WO 2010/007436 A2 (FIRST WATER LTD; MUNRO HUGH SEMPLE [GB]; ANDREWS PHILIP [GB]) 21 January 2010 (2010-01-21) * claims 1, 5; example 4 * ----- | 1-15 | |
| A | WO 2021/212154 A1 (KRATON POLYMERS LLC [US]) 21 October 2021 (2021-10-21) * claims 1-15; example 4 * ----- | 1-15 | |
| A | US 2019/151496 A1 (LAURENSOU CHRISTELLE [FR]) 23 May 2019 (2019-05-23) * paragraph [0030] - paragraph [0033]; claim 13 * * paragraphs [0041], [0063] * ----- | 1-15 | TECHNICAL FIELDS SEARCHED (IPC) A61L |

The present search report has been drawn up for all claims

1

| Place of search | Date of completion of the search | Examiner |
|---|---|---|
| The Hague | 13 May 2025 | Lopez Serrano, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 22 2210

This annex lists the patent family members relating to the patent documents cited in the above-mentioned European search report. The members are as contained in the European Patent Office EDP file on
The European Patent Office is in no way liable for these particulars which are merely given for the purpose of information.

13-05-2025

| Patent document cited in search report | Publication date | Patent family member(s) | Publication date |
|---|---|---|---|
| EP 4487876 A1 | 08-01-2025 | EP 4487876 A1 | 08-01-2025 |
| | | WO 2025012129 A1 | 16-01-2025 |
| US 2020040147 A1 | 06-02-2020 | BR 112019018566 A2 | 14-04-2020 |
| | | CN 110418815 A | 05-11-2019 |
| | | DE 102017105195 A1 | 13-09-2018 |
| | | DK 3592807 T3 | 13-09-2021 |
| | | EP 3592807 A2 | 15-01-2020 |
| | | EP 3872128 A1 | 01-09-2021 |
| | | ES 2887942 T3 | 29-12-2021 |
| | | ES 2961259 T3 | 11-03-2024 |
| | | JP 7632997 B2 | 19-02-2025 |
| | | JP 2020511220 A | 16-04-2020 |
| | | JP 2022009535 A | 14-01-2022 |
| | | US 2020040147 A1 | 06-02-2020 |
| | | US 2025066561 A1 | 27-02-2025 |
| | | US 2025066562 A1 | 27-02-2025 |
| | | WO 2018162009 A2 | 13-09-2018 |
| WO 2010007436 A2 | 21-01-2010 | EP 2313118 A2 | 27-04-2011 |
| | | US 2011190722 A1 | 04-08-2011 |
| | | US 2014249496 A1 | 04-09-2014 |
| | | WO 2010007436 A2 | 21-01-2010 |
| WO 2021212154 A1 | 21-10-2021 | AU 2021102033 A4 | 10-06-2021 |
| | | EP 4110409 A1 | 04-01-2023 |
| | | US 2023211041 A1 | 06-07-2023 |
| | | WO 2021212154 A1 | 21-10-2021 |
| US 2019151496 A1 | 23-05-2019 | AU 2008259660 A1 | 11-12-2008 |
| | | BR PI0811949 A2 | 11-11-2014 |
| | | CA 2687730 A1 | 11-12-2008 |
| | | CN 101678036 A | 24-03-2010 |
| | | DK 2148686 T3 | 28-07-2014 |
| | | EP 2148686 A2 | 03-02-2010 |
| | | ES 2494316 T3 | 15-09-2014 |
| | | FR 2916355 A1 | 28-11-2008 |
| | | HK 1138785 A1 | 03-09-2010 |
| | | JP 5460580 B2 | 02-04-2014 |
| | | JP 2010527673 A | 19-08-2010 |
| | | KR 20100016274 A | 12-02-2010 |
| | | PL 2148686 T3 | 31-10-2014 |
| | | PT 2148686 E | 02-09-2014 |
| | | RU 2009147168 A | 27-06-2011 |
| | | US 2010204174 A1 | 12-08-2010 |
| | | US 2016296658 A1 | 13-10-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 24 22 2210

This annex lists the patent family members relating to the patent documents cited in the above-mentioned European search report. The members are as contained in the European Patent Office EDP file on
The European Patent Office is in no way liable for these particulars which are merely given for the purpose of information.

13-05-2025

| Patent document cited in search report | Publication date | Patent family member(s) | Publication date |
| --- | --- | --- | --- |
| | | US 2019151496 A1 | 23-05-2019 |
| | | WO 2008149035 A2 | 11-12-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1718257 B1
- EP 1988909 A
- EP 2736486 A1
- EP 2742997 B1
- WO 2021198464 A1
- WO 2021212154 A1